Europäisches Patentamt

European Patent Office

Office européen des brevets

(11) Publication number: 0 225 407
A1

# EUROPEAN PATENT APPLICATION

(21) Application number: 85308747.6

(22) Date of filing: 02.12.85

(51) Int. Cl.⁴: C01B 33/20 , B01J 23/10 , C07C 1/20

(43) Date of publication of application:
16.06.87 Bulletin 87/25

(84) Designated Contracting States:
BE CH DE FR GB IT LI

(71) Applicant: Council of Scientific and Industrial Research
Rafi Marg
New Delhi 110 001(IN)

(72) Inventor: Ratnasamy, Paul
National Chemical Laboratory Pune - 4111 008
Maharashtra(IN)
Inventor: Kulkarni, Suneeta Balvant
National Chemical Laboratory Pune - 4111 008
Maharashtra(IN)
Inventor: Kotasthane, Arvind Narayan
National Chemical Laboratory Pune - 4111 008
Maharashtra(IN)
Inventor: Shiralkar, Vasudeo Pandurang
National Chemical Laboratory Pune - 4111 008
Maharashtra(IN)
Inventor: Kumar, Rajeev c/o Dr. Prof. J. Weitkamp
Engler Bunte Inst. der Karlsruhe TH
Postfach 6380 D-7500 Karlsruhe(DE)
Inventor: Hedge, Sooryakant Ganesh
National Chemical Laboratory Pune - 4111 008
Maharashtra(IN)
Inventor: Balakrishnan, Ikkandath
National Chemical Laboratory Pune - 4111 008
Maharashtra(IN)

(74) Representative: Spencer, Graham Easdale et al
A.A. Thornton & CO Northumberland House
303-306, High Holborn
London WC1V 7LE(GB)

(54) Lanthanum silicate materials, their production and use.

(57) Lanthanum silicate catalyst composites having, in terms of oxide mole ratios, the composition:

0 -0.3 $M_2O$ : $La_2O_3$ : 30-200 $SiO_2$

in which M is a monovalent cation. Such catalyst composites are made by reacting a lanthanum compound, an alkali metal compound and a silicon compound with a tetraalkyl ammonium salt of the formula:

$R^1_xR^2_yN^+Z$

in which $R^1$ and $R^2$ are $C_2$-$C_4$ alkyl groups, x and y are integers from 1 to 3 such that x + y is 4, and Z is a bromide or hydroxide ion, in an aqueous sulphuric acid medium to form a gel, heating the gel at a temperature of 100°C to 200°C, and filtering, drying and calcining the resulting solid material.

These materials are effective catalysts for the conversion of lower alcohols to lower olefins.

## Lanthanum silicate materials, their production and use

The present invention is concerned with certain novel lanthanum silicate materials and with their production and use. More particularly, this invention is concerned with novel lanthanum silicate materials which possess active properties as a catalyst composite and the use of such materials as a catalyst in the conversion of alkanols to olefins.

A number of catalysts for the conversion of alkanols, such as methanol, ethanol and propanol, to olefinic hydrocarbons have been described in the prior art. Thus, it is well known to employ catalysts such as alumina, silica-alumina and various aluminosilicates to convert ethanol to ethylene at elevated temperatures. Our European Patent Application No. 84302893.7 discloses the conversion of alkanols, including methanol, to olefins employing a crystalline encilite catalyst composite having, in terms of oxide mole ratios, the following composition:

$1.0 \pm 0.3$ $M_2O$ : $Fe_2O_3$ : 30-300 $SiO_2$ : $ZH_2O$

wherein M is a monovalent cation and Z is a value from 0 to 20.

The importance of olefinic hydrocarbons or olefins as they are more popularly called arises from their several applications in the chemical, petrochemical, plastics, synthetic fibre and detergent fields. The olefins which find particular use in these fields include the light olefins, particularly ethylene, propylene and butenes. These olefins are conventionally prepared by the thermal cracking of crude petroleum in the form of naphtha or gas oil in the presence of a catalyst for the cracking reaction. An alternative process for the production of light olefins comprises the catalytic conversion of alkanols. The manner in which or the source from which such alkanols are obtained is unimportant, although a convenient method is the conversion of coal or natural gas to synthesis gas comprising a mixture of carbon monoxide and hydrogen and the conversion of such synthesis gas by well-known techniques to alkanols.

Unfortunately, the production of olefins by the catalytic conversion of alkanols suffers from the serious limitation that the known catalysts hitherto employed for such conversion provide very low selectivity with respect to olefins from the conversion reaction. Furthermore, with respect to the conversion of a particular alkanol, that is methanol, there are no catalysts available commercially which are capable of bringing about the desired conversion in a single direct step with significant yields of the desired light olefins.

In view of what is stated above, the basic aim of the present invention is the provision of a novel catalytic material capable of being employed in the conversion of alkanols, particularly methanol, to olefin-rich products in a single direct step.

It has been established that the crystal and pore structure of metal silicate catalyst composites are influenced by the nature and concentration of the cations present in the solution from which the nucleation of the metal silicate composites is effected. The use of inorganic cations, such as sodium or potassium, and organic cations, such as tetrapropyl ammonium, is well known to influence the course and rate of crystallization of metal silicates. For example, it was found that the absence of such ammonium ions from the reaction medium during crystallization gave rise to a solid product which had negligible activity for the conversion of alcohols to hydrocarbons. Likewise, it was found that the presence of mixed alkyl ammonium ions alone in the reaction medium containing the oxides of the metal and silicon, without sufficient amounts of alkali metal cations, such as sodium or potassium ions, also failed to yield a product with the desired catalytic activity.

In the course of the investigation leading to the present invention, it was discovered that certain metal silicates, that is those of the rare earth family and more specifically silicates of lanthanum, when prepared using the starting materials and processes of preparation described hereinafter possess the surprising ability, not hitherto known, of converting methanol into olefins, especially ethylene, propylene and butenes.

According to the present invention there is provided a lanthanum silicate catalyst composite having, in terms of oxide mole ratios, the following composition:

0 -0.3 $M_2O$ : $La_2O_3$ : 30 -200 $SiO_2$

in which M is a monovalent cation.

The exact reason for the enhanced catalytic selectivity of the lanthanum silicates of this invention is the subject of speculation, but it is believed that the lower ratio of the charge to the mass of the trivalent lanthanum cation gives rise to a lower acidity of the associated protonic acidity of the composite compared to that of other metal silicates, such as alumino and iron silicates. This lower acidity presents an advantage in that while it is adequate to assist the conversion of alkanols to light olefins, it is insufficiently strong to convert the olefins so produced further to aromatic hydrocarbons and heavier aliphatics by hydrogen transfer reactions. Thus, the lower acidity enhances the selectivity of the lanthanum catalyst composite of the invention with respect to light olefins.

The present invention also comprises a process for the preparation of a lanthanum silicate catalyst composite according to the invention, which comprises reacting a lanthanum compound, an alkali metal compound, and a silicon compound with a tetraalkyl ammonium salt of the formula:

$R^1_xR^2_yN + Z$

in which $R^1$ and $R^2$, which may be the same or different, are alkyl groups containing from 2 to 4 carbon atoms; x and y, which may be the same or different, are values of from 1 to 3, with the sum of x and y equal to 4; and Z is a bromide or hydroxide ion, in an aqueous sulphuric acid medium to form a gel, heating the gel so formed at a temperature from 100°C to 200°C and thereafter filtering, drying and calcining the resultant solid composite material.

The preferred alkyl groups represented by $R^1$ and $R^2$ in the tetraalkyl ammonium salt are methyl, ethyl, propyl and butyl. Preferred tetraalkyl ammonium salts include, for example, triethyl-n-propyl ammonium bromide, triethyl-n-propyl ammonium hydroxide, triethyl-n-butyl ammonium bromide, and triethyl-n-butyl ammonium hydroxide.

The lanthanum compound used for the reaction may be, for example, a nitrate, sulphate, oxide or hydroxide of lanthanum in various structural and textural modifications.

Suitable silicon compounds are, for example, oxides of silicon in various physical modifications, such as colloidal silica, fumed silica, chromatographic silica, and silica gel.

The alkali metal compound used is preferably an alkali metal silicate and/or an alkali metal hydroxide. The preferred alkali metal is sodium.

Although the solid catalyst composite material of the present invention is referred to as a lanthanum silicate catalyst composite, it may contain a small amount of aluminium in addition to the lanthanum. The reason for this lies in the fact that those silicon compounds which, from an economic point of view, are used for the preparation of the lanthanum composite usually contain a small quantity of aluminium as an impurity. It is this aluminium which invariably finds its way, at least partly, into the final lanthanum silicate composite.

In the novel catalyst composites of the present invention, the monovalent cation M is preferably sodium, ammonium, or hydrogen.

According to a preferred feature of the invention, when M in the composition of the solid catalyst composite material is sodium, the calcined solid composite is subjected to ion exchange with an ammonium salt in order to replace sodium with ammonium ions and to thereby provide a product having a molar ratio of sodium oxide to aluminium oxide of from 0.01 to 0.3. Where the ammonium salt employed for the ion exchange is other than ammonium nitrate or ammonium chloride, the product resulting from the ion exchange is further treated with a mineral acid, such as nitric acid or hydrochloric acid, at a temperature of from 30°C to 90°C for a period of from 5 to 10 hours. Where the ammonium salt employed for ion exchange is ammonium nitrate or ammonium chloride, the further mineral acid treatment is not necessary, but the resulting product of the ion exchange should be washed free of chloride or nitrate ions before being dried and further calcined at about 540°C.

The gel obtained from the initial reaction is preferably heated to from 100°C to 180°C for 5 to 500 hours. The wet cake resulting from such heating is washed free of excess bromide ions, dried at approximately 120°C and calcined. The preferred calcination temperature is from 530°C to 550°C and calcination is effected over a period of from 6 to 10 hours.

According to yet another preferred feature of the invention, the various reactants in the process for producing the catalyst composite are reacted in stoichiometric amounts and the product is then crystallized hydrothermally to yield a catalyst composite composed of amorphous and/or crystalline lanthanum oxide, silica and lanthanum silicates, the molar ratio of sodium oxide and lanthanum oxide in said lanthanum silicates being at least 0.05 but not more than 0.3 and that of silica and lanthanum oxide being at least 25 but not more than 200, and the molar ratio of silica to lanthanum oxide in said amorphous and/or crystalline lanthanum oxide being at least 5 but not more than 200.

The lanthanum silicate solid catalyst composite of the present invention can be employed directly as a catalyst in alkanol-conversion reactions. However, in order to impart greater mechanical strength thereto, it is more convenient to blend the catalyst composite with one or more binders or carriers therefor, the binder preferably being used in an amount of from 10% to 70% by weight. Suitable binders or carriers for this purpose are, for example, alumina, silica, acetic acid, bentonite, kaolinite, talc, and stearic acid. After the composite and binder have been blended, the blend can be extruded to any desired shape, dried and calcined, suitably at a temperature of about 500°C for from 2 to 10 hours. The supported catalyst composite thus prepared is exceptionally useful in various processes of industrial interest, such as cracking, hydrocracking, isomerisation and alkylation of petroleum refinery streams, disproportionation, isomerisation and alkylation of aromatic hydrocarbons and, particularly, conversion of alkanols to light olefins.

In considering the efficiency and efficacy of the lanthanum silicate composites of the present invention as catalysts, the conclusion reached is that the presence of both sodium and mixed alkyl-ammonium ions is necessary in the reaction medium in addition to the oxides of silicon and lanthanum in order to obtain a solid material with the desired catalytic activity. Even though the precise mechanism by which alkyl-ammonium ions, when present in the reaction medium, influence the rate and course of the crystallisation of the desired catalytic mass is not known in detail, it is possible to speculate that they influence the structure of water in the vicinity of the reacting $SiO_4$ and lanthanum tetrahedral species in such a way that nucleation and crystallisation occur, leading to products with a desirable porous structure which are active, selective and stable in the conversion of alkanols, chiefly methanol, to olefins. Sodium ions which possess a much larger value of the charge to mass ratio and which occupy a much smaller volume in space, are perhaps not able to accomplish the desired structural rearrangements in the water phase as efficiently as the alkyl-ammonium ions. Likewise, aluminium ions which also possess a much larger value of the charge to mass ratio and occupy a much smaller volume in space are perhaps equally unable to accomplish the desired structural rearrangements in water phase with as much efficiency as either alkyl-ammonium ions or lanthanum ions. The characteristics of the pore structure of the solid catalyst composite of the present invention can be established from the amount of water, n-hexane and cyclohexane molecules that can be adsorbed in the pores of the composite at 24°C. While the extent of hydrophilicity of the pores is indicated by the amount of $H_2O$ molecules adsorbed, the adsorption of n-hexane can be used to evaluate the hydrophobic nature of the internal surface of the catalytic solid material. It was invariably found that for the catalyst composite of the present invention, the amount of n-hexane adsorbed per unit weight of solid was always higher than the amount of water adsorbed indicating a highly hydrophobic nature of the internal surfaces of the catalyst of the present invention. It is speculated that the unique catalytic activity of this material in the conversion of alkanols, such as methanol, to light olefins is due, at least in part, to this hydrophobic nature of the catalyst surface since water molecules formed as products during the conversion reactions will not be retained on the hydrophobic surface very strongly and hence will not block the access of methanol molecules to the active surface.

The unique catalytic properties of the lanthanum silicate composite of the present invention makes it an ideal catalyst for employment in the conversion of alkanols, such as methanol, to light olefins.

Accordingly, the present invention also provides a method for the conversion of alkanols to light olefins having from 2 to 4 carbon atoms, which comprises contacting one or more alkanols at a temperature of from 300°C to 650°C with a lanthanum silicate catalyst composite according to the invention or which has been produced by a process according to the invention.

The alkanols converted by this method may be in the form of an aqueous solution or in the vapor phase and the conversion can conveniently be effected at atmospheric pressure.

The lanthanum silicate catalyst composite is preferably provided within a fixed bed reactor and the aqueous alkanol solution or gaseous alkanol(s) is/are passed over the catalyst bed at a weight hourly space velocity of from 2 to 20. The alkanol most suited for conversion by the method of the invention to light olefins is methanol, but other alkanols having up to 4 carbon atoms may also be employed.

The framework vibration frequencies in cm$^{-1}$ of the lanthanum silicate catalyst composite of the present invention are set out below in Table 1.

## TABLE 1

### FRAMEWORK VIBRATION FREQUENCIES OF LANTHANUM SILICATE

$$SiO_2/La_2O_3 = 90$$

| Frequency cm$^{-1}$ | Intensity |
| --- | --- |
| 450 | VS |
| 550 | S |
| 595 | Sh |
| 625 | Sh |
| 685 | VW |
| 790 | S |
| 1000 | Sh |
| 1085 | VS,B |
| 1225 | Sh |

For appreciating the value of the intensity set out in Table 1, the following explanation is given:
VS -Very small
S -Strong
Sh -Shoulder
VW -Very weak
B -Broad

In order that the invention may be more fully understood, the following examples are given by way of illustration.

### Example 1

The example illustrates the preparation of the lanthanosilicate catalyst composite material. To 20 gms of sodium silicate solution (0.2% Na₂O, 27.2% SiO₂, 64.6% H₂O), 10 ml of water is added to constitute solution A. 2.5 gms of tetrapropylammonium bromide is dissolved in 15 ml of water to yield solution B. Solution C,is prepared by dissolving 0.65 gms of LaCl₃•7H₂O in 10 ml H₂O. 1.76 gms of H₂SO₄ (98%) is diluted in 15 ml water to yield solution D. Solution B is added to solution A with stirring. Solution C is then added to the mixture (A + B) with stirring and finally mixed with solution D, which is added dropwise while stirring vigorously. A free flowing gel is formed whose pH is adjusted to 10.2 ± 0.1. The gel is stirred for 15 minutes to obtain a uniform fluid slurry which is transferred to a stainless steel autoclave and the mass is heated to 180° ± 5 under autogeneous pressure for 35 hours. The contents of the autoclave are cooled to room temperature. The pH of the supernatent liquid which was obtained by filtration was 12.2 ± 0.2. The solid product was washed with hot water (deionized) till free from sulphate ions. The solid was then dried in air at 120°C for 12 hrs and then calcined at 540°C for 5 hrs to yield a solid material. The infrared framework vibration frequencies of the material are given in Table 1 above. The solid product so obtained free from organic matter was further exchanged twice with 5 l. ammonium chloride solution (free from Na) using 10 ml solution per gram for 16 hrs at 90°C for the 1st exchange (6 hrs for 2nd exchange). The product was filtered, washed with hot water to make it free from excess ammonium chloride. The product is dried in air at 120°C for 10 hrs and deammoniated/calcined at 550°C for 10 hrs to get protonic form. This product is further treated with 0.1 N nitric acid (5 ml/gm) for 6 hrs at 90°C. The product is then filtered, washed free from excess acid and calcined at 550°C for 10 hours.

The catalyst composite material so obtained is dry mixed with alumina (boehmite) binder (70:30) or with bentonite binder (65:15) on an anhydrous basis. The mixture is thoroughly dry mixed and water is added slowly till a thick lump is formed. It is then extruded using a stainless steel extruder of 1/16 inch (0.159 cm) diameter. Extrudates are air dried for 10 hrs at room temperature and calcined at 550°C for 10 hrs and then cooled to room temperature.

## Example 2

The procedure of Example 1 was repeated using the same starting materials except that triethyl-n-propyl ammonium bromide was used instead of tetrapropyl ammonium bromide. The solid material that was obtained exhibited framework vibration frequencies in the infrared region identical to those given in Table 1. The chemical composition of the calcined material was found to be

$(1.8 \pm 0.2)$ $Na_2O$ : $La_2O_3$ : $(90 \pm 5)$ $SiO_2 \cdot XH_2O$.

## Example 3

The silicate solution was made by mixing 800 gms of sodium silicate (27.2% $SiO_2$, 8.4% $Na_2O$ and 64.4% $H_2O$), 87.2 gms of diethyl dipropyl ammonium bromide, 400 gms of water. An acid solution was made by mixing 91.2 gm lanthanum chloride heptahydrate, 70.4 g $H_2SO_4$ and 1600 g of water. The acid solution was then added to a silicate solution slowly under vigorous stirring to form a lanthanosilicate gel. The gel had the following formula in the anhydrous form in terms of the mole ratio of oxides:

$4.38$ $R_2O$ : $25.55$ $Na_2O$ : $La_2O_3$ : $83$ $SiO_2$

where R is the diethyl ammonium cation.

The resulting gel was charged into a stainless steel autoclave of one gallon capacity and was maintained at 150°C without agitation for 130 hrs. The autoclave was cooled to room temperature and the solid product was separated from the supernatent liquid and washed with deionized water and dried at 120°C for 12 hrs, and calcined as illustrated in Examples 1 and 2 at 540°C. The IR data for this product was identical to that of the product set forth in Example 1.

## Example 4

A reaction mixture was prepared by combining 240 gms of sodium silicate (as in Example 1) and 300 gms of water to which was added a solution containing 30 gms of triethyl-n-butylammonium bromide - (TEBABr) dissolved in 100 gms of water and was stirred while adding an acid solution comprising of 92 gms of lanthanum chloride heptahydrate ($LaCl_3 \cdot 7H_2O$), 21.4 gms of 98% $H_2SO_4$, and 300 gms of water dropwise to obtain a free flowing slurry of pH $10.2 \pm 0.2$. The slurry was autoclaved at 180°C for a period of 36 hrs under autogeneous pressure. The autoclave was quenched in cold water and the solid reaction product was separated from the mother liquor by filtration and was washed with deionized water till free from sulphate and other soluble impurities. The solid was then dried in air at $120 \pm 5$°C for 12 hrs and then calcined at 550°C for 8 hrs to yield a solid crystalline lanthanosilicate having a $SiO_2/La_2O_3$ mole ratio of 90.

## Example 5

A lanthanosilicate gel was prepared from 200 gms of sodium silicate, 25 gms of TPABr, 16.7 gms of $H_2SO_4$ and 11.4 gms of $LaCl_3 \cdot 7H_2O$ and 500 gms of water, with constant stirring in the manner described in Examples 1 to 4 above. The gel was transferred to and sealed in a stainless steel pressure vessel and heated in an air oven at 150°C at autogeneous pressure for 48 hrs. The solid reaction product was recovered by filtration, washed with hot deionized water, dried in air at 120°C for 12 hrs. The IR absorption spectra of this product was identical with the product of Examples 1 and 2 and no other crystalline impurity was present. The $SiO_2/La_2O_3$ mole ratio was $90 \pm 5$.

7

## Example 6

This example illustrates the process of replacing the sodium ions in the lanthanosilicates by ammonium ions. A sample of the product of Example 1 was subject to ion exchange. The solid is twice treated with an aqueous solution of 5M $NH_4Cl$, at 90°C for 15 hours each time. After each exchange, the product is washed with hot distilled water until the filtrate was free from Cl ions. The product is dried at 120°C in an air oven for 12 hrs to yield the ammonium form of the lanthanosilicate. The molar ratio of $Na_2O/La_2O_3$ in the material was 0.04. By varying the concentration of $NH_4Cl$ and the number of treatments, the $Na_2O/La_2O_3$ ratio could be varied from 0.3 to 0.03.

## Example 7

This example illustrates the process of replacing $NH_4^+$ ions in the lanthanosilicate illustrated in Example 6 by protons ($H^+$). Samples of the product of Example 6 were calcined at 540°C for 10 hrs in a stream of dry air to yield the protonated or acid form of the lanthanosilicate.

## Example 8

This example illustrates the process for acid extraction of the lanthanosilicate described in Example 7 to remove part of the $La_2O_3$ occluded in the pores of the lanthanosilicate. 50 gms of the protonated form is treated with 250 ml of 0.1N $HNO_3$ for 6 hrs at 90°C with intermittent stirring of the acid slurry. The slurry is filtered and washed free of $NO_3$ ions with hot deionized water. Filtrate analysis shows that a portion of $La_2O_3$ was extracted out of the lanthanosilicate by this treatment. The product is dried at 120°C and calcined at 540°C for 2 hrs to yield the active form of the catalyst.

## Example 9

Lanthanosilicate catalyst extrudates with increased mechanical strength were prepared by treating and mixing the hydrogen form of the lanthanosilicate powder described in Example 8 above with alumina binder at room temperature and shaping the mass by passing through a screw-driven steel die of 1/8 inch (0.318 cm) opening. The content of $Al_2O_3$ binder was varied from 15% to 50% on a dry basis. The shaped extrudates were kept in the atmosphere for 2 to 4 hrs and dried in an air oven at 100-110°C for about 4 hours and finally calcined in a muffle furnace at 550°C for 10 hrs.

The calcined extrudates were treated with 0.1 N HCl on a water-bath, filtered and washed free of chloride ions. The acid treated extrudates have improved activity and selectivity for the formation of $C_2$-$C_4$ olefins from methanol.

## Example 10

Using the same mixing sequence as described in Example 1 and the same reaction components except that the quaternary ammonium compound was tri-n-propyl ethyl ammonium bromide, a gel was formed by mixing the following reaction components in the following molar ratios on an oxide basis:

4.38 $R_2O$: 25.5 $Na_2O$: $La_2O_3$: 83 $SiO_2$: 3365 $H_2O$

where R is the tri-n-propyl ethyl ammonium ion. The thick homogeneous gel was autoclaved at 180°C without agitation for 36 hrs. The solid product was filtered, washed and dried as described in Examples 1 to 5. The product exhibited a similar IR pattern confirming it to be a similar crystalline lanthanosilicate.

## Example 11

This example illustrates the preparation of lanthanosilicate using fumed silica as a starting material instead of sodium silicate. The reactant gel was prepared by mixing the following components:

1. Fumed silica 12 gms
2. NaOH 1.27 gms

3. TPABr 2.73 gms

4. $LaCl_3 \cdot 7H_2O$ 0.57 gms

5. Water 75 ml

The resulting homogeneous gel was charged into a stainless steel autoclave. Crystallization was carried out at 180°C under autogeneous pressure and the reaction product was filtered, washed, dried and calcined as described in Example 1.

The lanthanosilicate showed a similar IR pattern to that in Table 1.


## Example 12

Methanol was passed in the vapour phase at atmospheric pressure over a catalyst according to the invention which was contained in a fixed bed reactor, at a weight hourly space velocity (WHSV) of 2.2, WHSV being the weight in grams of the reactant, in this case methanol, which was passed over one gram of the catalyst per hour at the specified temperatures and pressures mentioned below. The products of the reaction were analysed quantitatively by gas chromatography. The results are shown in Table 2. The term "Product" is used in this example and in the following examples to refer to the hydrocarbon products of the reaction.


## Table 2

### Conversion of methanol to hydrocarbons

| | |
|---|---|
| Feed | : 100% methanol |
| WHSV ($hr^{-1}$) | : 2.2 |
| Pressure | : atmospheric |
| Temperature | : 450°C |
| % conv: of methanol | : 99 |
| DME (dimethylether) wt% | : Trace |
| Product distribution (wt %) | : |
| Ethylene | : 11.1 |
| Propylene | : 37.6 |
| Butenes | : 20.5 |
| Total $C_2$ to $C_4$ olefins | : 69.2 |
| Total $C_1$ to $C_4$ saturates | : 9.0 |
| $C_5$ + hydrocarbons | : 21.8 |


## Example 13

This example illustrates the effect of dilution of the feed methanol with water. It also shows that even dilute solutions of methanol can be used for conversion of methanol to olefins with advantage. The experiment and analysis of products were carried out as described in Example 12; Table 3 presents the experimental data.

## Table 3

### Conversion of aqueous methanol to olefins

| | |
|---|---|
| Feed (w/w) | : 40% methanol |
| WHSV ($hr^{-1}$) | : 2.16 |
| Pressure | : atmospheric |
| Temperature | : 450°C |
| % conv: of methanol | : 90 |
| DME | : 9.3 |
| Product distribution (wt %) | |
| Ethylene | : 12.46 |
| Propylene | : 58.21 |
| Butenes | : 10.61 |
| Total $C_2$ to $C_4$ olefins | : 81.2% |
| Methane | : 0.97 |
| Ethane | : Trace |
| Propane | : 0.61 |
| Butanes | : 1.27 |
| $C_1$ to $C_4$ saturates | : 2.85 |
| $C_5$ + hydrocarbons | : 15.87 |

Example 14

This example illustrates the effect of temperature on the conversion of an aqueous solution of methanol. The experiment and analysis of products were carried out as described in Example 12; the results are presented in Table 4.

## Table 4

### Conversion of aqueous methanol solution to olefins - effect of temperature

Feed : 80% (w/w) :

WHSV (hr$^{-1}$) : 4.32

| | | $420^{\circ}C$ | $450^{\circ}C$ | $480^{\circ}C$ |
|---|---|---|---|---|
| Pressure : atmospheric | : | | | |
| % conv: of methanol | : | 98.0 | 98.5 | 98.3 |
| DME | : | <1.0 | <1.0 | <1.0 |
| Product distribution (wt %) | | | | |
| Ethylene | : | 11.52 | 13.53 | 14.58 |
| Propylene | : | 39.50 | 53.31 | 48.58 |
| Butenes | : | 14.50 | 15.90 | 15.34 |
| Total $C_2$ to $C_4$ olefins | : | 65.92 | 82.74 | 78.77 |
| Methane | : | 0.31 | 0.61 | 0.70 |
| Ethane | : | Trace | Trace | Trace |
| Propane | : | 1.10 | 1.65 | 1.63 |
| Butanes | : | 3.39 | 2.84 | 2.11 |
| $C_1$ to $C_4$ saturates | : | 4.80 | 5.10 | 4.44 |
| $C_5$ + hydrocarbons | : | 29.28 | 12.16 | 15.79 |

### Example 15

This example illustrates the effect of the extent of dilution of methanol with water on product distribution in the conversion of methanol to olefins. It further confirms that aqueous solutions of methanol can be converted to olefins with advantage. Table 5 shows the experimental data for various dilutions of feed methanol. The experimental set up and analysis of products are as described earlier.

11

## Table 5

| Feed : (w/w% methanol) | : | 100 | 80 | 60 | 40 |
|---|---|---|---|---|---|
| WHSV (hr$^{-1}$):4.32 | | | 5 | | |
| Pressure : | : | atmospheric | | | |
| Temperature | : | 485°C | | | |
| conv: of methanol | : | 97.21 | 99.5 | 99 | 89.32 |
| DME | : | <1.0 | t | t | 3.3 |
| Product distribution (wt.%) | | | | | |
| Ethylene | : | 12.31 | 12.76 | 12.57 | 12.44 |
| Propylene | : | 41.38 | 42.42 | 45.26 | 46.51 |
| Butenes | : | 19.28 | 19.98 | 18.47 | 18.02 |
| Total $C_2$ to $C_4$ olefins | : | 72.97 | 75.16 | 76.60 | 76.97 |
| Methane | : | 0.83 | 0.61 | 0.66 | 0.65 |
| Ethane | : | 0.10 | 0.10 | 0.10 | - |
| Propane | : | 2.43 | 1.57 | 1.09 | 0.81 |
| Butanes | : | 3.23 | 2.37 | 1.61 | 1.33 |
| $C_1$ to $C_4$ saturates | : | 6.59 | 4.55 | 3.36 | 2.79 |
| $C_5$ + hydrocarbons | : | 10.44 | 20.29 | 19.94 | 20.20 |

(t = trace)

### Example 16

The effect of space velocity is illustrated in this example. The feed used was 80% aqueous methanol solution. The procedure for carrying out the experiment and analysis of products is as described in Example 12; Table 6 presents the data for various space velocities.

12

## Table 6

### Conversion of methanol to olefins - effect of space velocity

| Feed (w/w) : 80% methanol | : | | | | |
|---|---|---|---|---|---|
| WHSV (hr$^{-1}$) | : | 1.60 | 5.1 | 12.75 | 27.5 |
| Pressure : atmospheric | : | | | | . |
| Temperature | : | | $-480^{\circ}C-$ | | |
| % conv: of methanol | : | 100 | 97 | 97 | 72.5 |
| DME | : | nil | 2 | 2 | 23.5 |
| Product distribution, wt % | | | | | |
| Ethylene | : | 14.60 | 10.49 | 8.42 | 7.38 |
| Propylene | : | 39.59 | 51.48 | 51.19 | 40.63 |
| Butenes | : | 23.66 | 14.05 | 13.41 | 12.44 |
| Total $C_2$ to $C_4$ olefins | : | 78.85 | 76.02 | 73.02 | 60.45 |
| Methane | : | 1.87 | 0.46 | 0.46 | 0.72 |
| Ethane | : | 0.37 | t | t | t |
| Propane | : | 1.54 | 0.86 | 0.49 | 0.26 |
| Butanes | : | 1.70 | 1.39 | 1.11 | 0.75 |
| $C_1$ to $C_4$ saturates | : | 5.48 | 2.75 | 2.06 | 1.73 |
| $C_5$ + hydrocarbons | : | 16.44 | 21.25 | 24.92 | 37.82 |

### Example 17

This example illustrates the effect of time on stream on the activity and product distribution in the conversion of 80% aqueous methanol solution to olefins. The experimental technique and analysis of products were as described in Example 12; Table 7 presents the run data.

0 225 407

### Table 7

Conversion of aqueous methanol to olefins
effect of time on stream

| Feed (w/w) | : | 80% | | | |
|---|---|---|---|---|---|
| $WH_3V$ ($hr^{-1}$) | : | 5.1 | | | |
| Pressure | : | atmospheric | | | |
| Temperature | : | $450^{\circ}C$ | $480^{\circ}C$ | $480^{\circ}C$ | $480^{\circ}C$ |
| % conv: of methanol (wt %): | | 20 | 40 | 70 | 80 |
| DME | : | 94 | 96 | 95 | 94 |
| Product distribution, wt % | | | | | |
| Ethylene | : | 10.8 | 13.4 | 11.6 | 11.4 |
| Propylene | : | 46.6 | 49.8 | 49.6 | 49.1 |
| Butenes | : | 15.4 | 13.7 | 14.1 | 13.4 |
| Total $C_2$ to $C_4$ olefins | : | 72.8 | 76.9 | 75.3 | 73.8 |
| Methane | : | 0.4 | 0.7 | 0.9 | 0.9 |
| Ethane | : | t | t | t | t |
| Propane | : | 1.2 | 1.0 | 0.8 | 0.8 |
| Butanes | : | 2.4 | 1.4 | 1.3 | 1.2 |
| $C_1$ to $C_4$ saturates | : | 4.0 | 3.0 | 3.0 | 2.8 |
| $C_5$ + hydrocarbons | : | 23.2 | 20.1 | 21.7 | 23.4 |

(t = trace)

#### Example 18

The catalyst according to the invention can also be used in the form of extrudates after mixing with any suitable binder, for example bentonite, prior to extrusion. The performance of such extrudates as catalyst is similar to that shown in Examples 12-17, as illustrated by the data in Table 8. A higher temperature should be used, however, in order to obtain the maximum yields of olefins. Table 8 also illustrates the effect of time on stream on the performance of the catalyst.

14

## Table 8

### Conversion of aqueous methanol to olefins using extruded catalysts

Feed : 40% methanol :

WHSV : 2.55 :

Pressure : atmospheric :

Temperature : 450°C :

| % conv: of methanol (wt %) | : | 97 | 97 | 97 | 98 | 97 | 97 | 95 |
|---|---|---|---|---|---|---|---|---|
| DME (wt %) | : | ⟨1 | ⟨1 | 1 | 1 | 1 | 1 | 1 |
| Time on stream (hr) | : | 4 | 16 | 22 | 28 | 34 | 44 | 47 |
| Product distribution, wt % | : | | | | | | | |
| Ethylene | : | 13.0 | 12.7 | 12.2 | 13.0 | 11.5 | 14.6 | 9.0 |
| Propylene | : | 43.3 | 42.6 | 43.7 | 47.0 | 48.3 | 52.9 | 45.9 |
| Butenes | : | 18.7 | 18.9 | 17.6 | 18.8 | 18.4 | 11.3 | 14.6 |
| Total $C_2$ to $C_4$ olefins | : | 75.0 | 74.2 | 73.5 | 78.8 | 78.2 | 78.9 | 69.5 |
| Methane | : | 0.8 | 1.0 | 1.1 | 1.1 | 1.1 | 1.8 | 1.8 |
| Ethane | : | t | t | t | t | – | – | t |
| Propane | : | 1.4 | 1.3 | 1.3 | 1.3 | 1.1 | 1.1 | 0.8 |
| Butanes | : | 1.7 | 1.8 | 2.3 | 2.6 | 2.7 | 2.2 | 2.2 |
| $C_1$ to $C_4$ saturates | : | 3.9 | 4.1 | 4.7 | 5.0 | 5.0 | 5.1 | 4.3 |
| $C_5$ + hydrocarbons | : | 21.1 | 21.6 | 21.8 | 18.2 | 16.8 | 16.0 | 26.4 |

(t = trace)

Example 19

This example illustrates that in place of aqueous methanol, a mixture of dimethyl ether and methanol can also be used to produce olefins in good yield. A double reactor system is used in which the methanol passes over a dehydration catalyst, such as alumina, in the first reactor to produce DME, the yield of which is determined by the methanol composition, temperature and flow rate employed. The DME and unconverted methanol are then passed over a catalyst according to the invention at temperature, WHSV, etc., as required to give the maximum yield of olefins. Typical data obtained from a representative run are presented in Table 9.

Table 9

Conversion of a mixture of DME + methanol to olefins

Feed (w/w) : 40% methanol

WHSV ($hr^{-1}$) : 2.55

Pressure : atmospheric

Temperature : $510^{\circ}C$

| | | |
|---|---|---|
| Conversion of methanol (wt %) | : | 99 + |
| DME (wt %) | : | Trace |
| Product distribution, wt % | | |
| Ethylene | : | 16.32 |
| Propylene | : | 48.53 |
| Butenes | : | 19.79 |
| Total $C_2$ to $C_4$ olefins | : | 84.64 |
| Methane | : | 0.94 |
| Ethane | : | 0.07 |
| Propane | : | Trace |
| Butanes | : | Trace |
| $C_1$ to $C_4$ saturates | : | 1.01 |
| $C_5$ + hydrocarbons | : | 14.33 |

Example 20

The effect of variation of the reaction temperature for the conversion of a mixture of methanol, DME and water, obtained from the first reactor, in a double reactor system as described in the previous example is illustrated in this example and data obtained are presented in Table 10. The experimental set up is the same as that of Example 19 and analysis of the products is as stated in Example 12.

## Table 10

### Conversion of mixture of methanol + DME + water-effect of temperature

Feed (w/w) : 30% methanol
WHSV ($hr^{-1}$)
Pressure : atmospheric

| | | | | | | |
|---|---|---|---|---|---|---|
| Temperature | : | $300^{\circ}C$ | $350^{\circ}C$ | $410^{\circ}C$ | $450^{\circ}C$ | $575^{\circ}C$ | $570^{\circ}C$ |
| Conversion of methanol (wt %) | : | 58.32 | 90.35 | 98.51 | 99+ | 99+ | 99+ |
| DME (wt %) | : | 56.75 | 7.55 | <1 | trace | nil | nil |
| Product distribution (wt %) | | | | | | | |
| Ethylene | : | 38.60 | 22.73 | 10.81 | 9.29 | 15.15 | 22.10 |
| Propylene | : | 29.20 | 17.89 | 28.41 | 41.31 | 47.45 | 46.42 |
| Butenes | : | 9.0 | 16.74 | 15.47 | 23.73 | 18.32 | 12.91 |
| Total $C_2$ to $C_4$ olefins | : | 76.80 | 57.36 | 54.69 | 74.53 | 80.92 | 81.43 |
| Methane | : | 1.9 | 0.58 | 0.80 | 0.51 | 1.00 | 2.62 |
| Ethane | : | trace | trace | trace | trace | trace | trace |
| Propane | : | 2.4 | trace | 2.4 | 1.76 | 1.69 | 1.11 |
| Butanes | : | 2.5 | trace | 6.24 | 3.99 | 2.21 | 0.83 |
| $C_1$ to $C_4$ saturates | : | 6.8 | trace | 9.44 | 6.26 | 4.90 | 4.56 |
| $C_5$ + hydrocarbons | : | 16.4 | 42.06 | 35.67 | 19.39 | 14.17 | 14.00 |

0 225 407

Example 21

This example illustrates the regeneratability of a catalyst which was coked and showed poor activity for conversion of aqueous methanol to olefins. The results obtained with the catalyst before and after coking and after regeneration of the catalyst are shown in Table 11. The experimental technique and analysis of the products are carried out as stated in Example 12.

### Table 11

Conversion of methanol to olefins, Comparison of data for fresh catalyst, coked catalyst and regenerated catalyst

| | | | |
|---|---|---|---|
| Feed (w/w) : 80% methanol | : | | |
| WHSV | : | 2.55 | 2.55 | 2.55 |
| Pressure : atmospheric | | | |
| Temperature | : | $510^{\circ}C$ | $510^{\circ}C$ | $80^{\circ}C$ |
| Conversion of methanol (wt %): | | 99 + | 93.41 | 99 |
| DME (wt %) | : | trace | 3.18 | $\simeq 1$ |
| Product distribution (wt %) | : | | | |
| Ethylene | : | 16.32 | 12.87 | 11.71 |
| Propylene | : | 48.53 | 44.29 | 48.40 |
| Butenes | : | 19.79 | 14.22 | 21.15 |
| Total $C_2$ to $C_4$ olefins | : | 84.64 | 71.38 | 81.26 |
| Methane | : | 0.94 | 2.66 | 0.95 |
| Ethane | : | 0.07 | t | t |
| Propane | : | | 0.65 | 0.80 |
| Butanes | : | | 0.73 | 1.42 |
| $C_1$ to $C_4$ saturates | : | | 4.03 | 3.17 |
| $C_5$ + hydrocarbons | : | 14.33 | 24.50 | 15.57 |

(t = trace)

## Claims

1. A lanthanum silicate catalyst composite having, in terms of oxide mole ratios, the following composition:
0 -0.3 $M_2O$ : $La_2O_3$ : 30 -200 $SiO_2$
in which M is a monovalent cation.

2. A lanthanum silicate catalyst composite having, in terms of oxide mole ratios, the following composition:
0 -0.3 $M_2O$ : $La_2O_3$ : 90 $SiO_2$
in which M is a monovalent cation.

3. A lanthanum silicate catalyst composite according to claim 1 or 2, in which M is sodium, ammonium or hydrogen.

4. A process for the preparation of a lanthanum silicate catalyst composite according to any of claims 1 to 3, which comprises reacting a lanthanum compound, an alkali metal compound and a silicon compound with a tetraalkyl ammonium salt of the formula:

$R^1_x R^2_y N^+ Z$

in which $R^1$ and $R^2$, which may be the same or different, are alkyl groups containing from 2 to 4 carbon atoms; x and y, which may be the same or different, are values of from 1 to 3 but with the sum of x and y equal to 4; and Z is a bromide or hydroxide ion,

in an aqueous sulphuric acid medium to form a gel, heating the gel so formed at a temperature from 100°C to 200°C and thereafter filtering, drying and calcining the resultant solid composite material.

5. A process according to claim 4, in which $R^1$ and $R^2$ in the formula $R^1_x R^2_y N^+ Z$ are methyl, ethyl, propyl or butyl.

6. A process according to claim 4 or 5, in which, when M in the composition for the composite is sodium, the calcined composite is subjected to ion exchange with an ammonium salt to provide a composite having a molar ratio of sodium oxide to aluminium oxide of from 0.01 to 0.3.

7. A process according to claim 6, in which the product resulting from said ion exchange is further treated with nitric acid or hydrochloric acid at a temperature of from 30°C to 90°C for a period of from 5 to 10 hours.

8. A process according to claim 6, in which the ammonium salt is ammonium nitrate or ammonium chloride and the product resulting from the ion exchange is washed to free it of chloride or nitrate ions before being dried and further calcined.

9. A process according to any of claims 4 to 8, in which the gel is heated to a temperature of from 150°C to 180°C for from 5 to 500 hours.

10. A process according to any of claims 4 to 9, in which the product resulting from the heating of the gel is washed, dried at approximately 120°C and calcined.

11. A process according to any of claims 4 to 10, in which the calcination or further calcination is effected at a temperature of from 530°C to 550°C over a period of from 6 to 10 hours.

12. A process according to any of claims 4 to 11, in which the reactants are reacted in stoichiometric amounts and the product is crystallised hydrothermally to yield a catalyst composite composed of amorphous and/or crystalline lanthanum oxide, silica and lanthanum silicates, the molar ratio of sodium oxide and lanthanum oxide in the lanthanum silicates being from 0.05 to 0.3 and that of silica and lanthanum oxide being from 25 to 200, and the molar ratio of silica to lanthanum oxide in the lanthanum oxide being from 5 to 200.

13. A process according to any of claims 4 to 12, in which the solid catalyst composite is blended with from 10% to 70% by weight of a binder, the blend is extruded to a desired shape, dried and calcined at a temperature of about 500°C for from 2 to 10 hours.

14. A process according to claim 13, in which the binder is alumina, silica, acetic acid, bentonite, keolinite, talc, or stearic acid.

15. A method for the conversion of alkanols to light olefins having from 2 to 4 carbon atoms, which comprises contacting one or more alkanols at a temperature of from 300°C to 650°C with a lanthanum silicate catalyst composite according to any of claims 1 to 3 or which has been produced by a process according to any of claims 4 to 14.

16. A method according to claim 15, in which the alkanol(s) is/are in the form of an aqueous solution or in the vapor phase and the contacting is effected at atmospheric pressure.

17. A method according to claim 15 or 16, in which the lanthanum silicate catalyst composite is provided within a fixed bed reactor and the aqueous alkanol solution or gaseous alkanol(s) is/are passed over the catalyst bed at a weight hourly space velocity of from 2 to 20.

18. A method according to any of claims 15 to 17, in which the contacting is effected at a temperature of from 300°C to 650°C.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 9, no. 12 (C-261)[1735], 18th January 1985; & JP - A - 59 164 391 (AJIA SEKIYU K.K.) 17-09-1984 | 1,2 | C 01 B 33/20<br>B 01 J 23/10<br>C 07 C 1/20 |
| X | PATENT ABSTRACTS OF JAPAN, vol. 7, no. 19 (C-147)[1164], 25th January 1983; & JP - A - 57 175 722 (NIPPON GOSEI GOMU K.K.) 28-10-1982 | 1,2,15 | |
| A | GB-A-1 433 885 (ORDENA LENINA INSTITUT KHIMICHESKOI FIZIKI) * example 2 * | 15 | |
| A | US-A-4 161 463 (MYERS et al.) * claims 1, 3 * | 1 | TECHNICAL FIELDS SEARCHED (Int. Cl.4) |
| A | FR-A-2 527 596 (SNAMPROGETTI SPA.) | | C 01 B 33/00<br>B 01 J 23/00<br>C 07 C 1/20 |

-----

The present search report has been drawn up for all claims

| Place of search | Date of completion of the search | Examiner |
|---|---|---|
| BERLIN | 24-06-1986 | CLEMENT J.P. |